# EUROPEAN PATENT APPLICATION

(11) **EP 4 559 388 A1**
(43) Date of publication of application: **28.05.2025**
(21) Application number: 23843305.6
(22) Date of filing: 17.07.2023
(51) Int. Cl.: A61B 5/055, A61B 5/00, G16H 50/20, G16H 30/40, G06N 3/08, G06N 3/045

(54) **BRAIN MRI ANALYSIS METHOD AND DEVICE USING NEURAL NETWORK**

(30) Priority: 18.07.2022 KR 20220088514; 04.07.2023 KR 20230086668
(71) Applicant: Seoul National University Hospital, Seoul 03080 (KR)
(72) Inventor: KIM, Joonghee, Seongnam-si, Gyeonggi-do 13620 (KR)
(74) Representative: V.O.
(86) International application number: PCT/KR2023/010212
(87) International publication number: WO 2024/019458

(57) **Abstract**

A brain magnetic resonance imaging (MRI) analysis device and method using a neural network are disclosed. The brain MRI analysis device using a neural network according to one embodiment comprises: a memory for storing a neural network model; and a processor, which is connected to the memory so as to control an analysis device, wherein the processor receives one or more brain MRI images so as to generate input data, and inputs the input data into the neural network model so as to acquire output data, and the neural network model is trained to determine neurological prognosis of a cardiac arrest patient if the input data is input into the neural network model.

## Description

### Technical Field

The disclosed embodiments relate to techniques for analyzing brain magnetic resonance imaging (MRI) using neural network.

### Cross-References to Related Applications

This application claims priority to Korean Provisional Patent Application No. 10-2022-0088514 filed on Jul. 18, 2022 and Korean Patent Application No.10-2023-0086668 filed on Jul. 4, 2023, the entire contents of which are incorporated into this application.

### Background

Cardiac Arrest is an emergency medical situation with a very low chance of survival as well as neurological recovery. Providing immediate basic life support (BLS) and optimal resuscitation may prevent cardiac arrest, but it is difficult to expect good neurological results. Thus, among other things, the most important thing for cardiac arrest patients is accurate prognostic determination.

In this regard, various neural prognostic determination methods exist, such as brain imaging, electroencephalography (EEG), somatosensory evoked potentials (SSEP), and serum biomarkers. Among them, brain magnetic resonance imaging (MRI), particularly diffusion weighted image (DWI), is known to be useful in determining the prognosis of cardiac arrest patients in coma. In addition, in order to improve accuracy, a method of utilizing various quantitative measurements of apparent diffusion coefficient (ADC) values of brain tissue also has been previously proposed.

However, their clinical use has limitations of low practicality using complex methods that are often difficult to implement. For example, their clinical use requires a region of interest (ROI) directly hand-marked by a user, including a clinician, or requires dedicated software or algorithms for ROI selection.

In order to improve the above limitations, some have attempted to utilize artificial intelligence in determining the prognosis of cardiac arrest patients thanks to the development of deep learning.

However, even in this case, there is a limitation in that it is difficult to obtain training data due to a low recovery rate of consciousness of a cardiac arrest patient.

### Detailed Description

### Technical Problems

The disclosed embodiments are for analyzing brain magnetic resonance imaging (MRI) using neural network.

### Technical Solution

A brain magnetic resonance imaging (MRI) analysis device using a neural network according to one embodiment includes: a memory for storing a neural network model; and a processor, which is connected to the memory so as to control the analysis device, wherein the processor receives one or more brain MRI images so as to generate input data, and inputs the input data into the neural network model so as to acquire output data, and wherein the neural network model is trained to determine neurological prognosis of a cardiac arrest patient if the input data is input into the neural network model.

The one or more brain MRI images may include at least one of a diffusion weighted image (DWI) image, an apparent diffusion coefficient (ADC) image, and a fractional anisotropy (FA) image.

The processor may generate each of the DWI image, the ADC image, and the FA image as a three-dimensional array of a preset size, and, when there are a plurality of types of the input brain MRI images, may generate the input data by concatenating a plurality of arrays of the DWI array, the ADC array, and the FA array along a new axis.

The neural network model may be convolution neural network (CNN) model including a plurality of successive 3D-CNN.

The neural network model may be a convolution neural network in which each of the plurality of 3D-CNNs is successively connected in the order of a normalization layer, an activation function, and a pooling layer after a convolution layer, followed by a fully connected layer (FC layer).

The neural network model may be supervised and trained from training data consisting of a brain MRI image labeled as a brain MRI image of a cardiac arrest patient and a brain MRI image labeled as a brain MRI image of a non-cardiac arrest patient.

A brain magnetic resonance imaging (MRI) analysis method using a neural network according to one embodiment, performed in a brain MRI analysis device using a neural network comprising: one or more processors, and a memory storing one or more neural network models executed by the one or more processors, the method includes: receiving one or more brain MRI images so as to generate input data; and inputting the input data into the neural network model so as to output the output data, wherein the neural network model is trained to determine neurological prognosis of a cardiac arrest patient if the input data is input into the neural network model.

The one or more brain MRI images may include at least one of a diffusion weighted image (DWI) image, an apparent diffusion coefficient (ADC) image, and a fractional anisotropy (FA) image.

The processor may generate each of the DWI image, the ADC image, and the FA image as a three-dimensional array of a preset size, and when there are a plurality of types of the brain MRI images, may generate the input data by concatenating a plurality of arrays of the DWI array, the ADC array, and the FA array along a new axis.

The neural network model may be convolution neural network (CNN) model including a plurality of successive 3D-CNN.

The neural network model may be a convolution neural network in which each of the plurality of 3D-CNNs is successively connected in the order of a normalization layer, an activation function, and a pooling layer after a convolution layer, followed by a fully connected layer (FC layer).

The neural network model may be supervised and trained from training data consisting of a brain MRI image labeled as a brain MRI image of a cardiac arrest patient and a brain MRI image labeled as a brain MRI image of a non-cardiac arrest patient.

A brain magnetic resonance imaging (MRI) analysis device using a plurality of neural network models according to one embodiment includes a memory for storing a plurality of neural network models; and a processor, which is connected to the memory so as to control the analysis device, wherein the processor receives input data comprising main data and/or auxiliary data, and performs one or more tasks for the input data to determine prognosis of a cardiac arrest patient by using a pre-trained model comprising a first neural network model, a second neural network model, and a third neural network model, wherein the first neural network model is trained to generate a feature vector for the main data, wherein the second neural network model is trained to perform one or more tasks for the main data, and wherein the third neural network model is trained to perform one or more tasks for the main data and/or auxiliary data.

When the input data includes main data, the second neural network model may be trained to perform one or more tasks for the input data, and when the input data includes auxiliary data, the third neural network model may be trained to perform one or more tasks for the input data.

The processor may, when the input data includes only the main data, perform one or more tasks for the input data by using the first neural network model and the second neural network model, and, when the input data includes the auxiliary data, perform one or more tasks for the input data by using the first neural network and the third neural network models.

The main data may be data generated from one or more brain MRI images of a cardiac arrest patient or a non-cardiac arrest patient.

The auxiliary data may be data generated from data comprising at least one of age information, gender information, blood test result information, cardiac arrest duration information, cardiac arrest cause information, body temperature information, consciousness information, and MRI equipment characteristic information of each of the cardiac arrest patient and the non-cardiac arrest patient.

The second neural network model and the third neural network model may be trained to analyze, as the one or more tasks, the input data with respect to at least one of a probability of death within a predetermined period, a probability of recovery of neurological function at a specific level or higher within the predetermined period, a lesion presence/absence probability, presence/absence of a specific lesion at a specific location, a probability of presence/absence of a specific disease, and an auxiliary task.

The second neural network model and the third neural network model may be trained to perform classification or regression analysis on the input data based on a type of a task performed on the input data.

A brain magnetic resonance imaging (MRI) analysis method using a neural network according to one embodiment, performed in a brain MRI analysis device using a neural network comprising: one or more processors, and a memory for storing one or more neural network models executed by the one or more processors, the method includes: receiving input data comprising main data and/or auxiliary data; and performing one or more tasks for the input data to determine a prognosis of a cardiac arrest patient by using a pre-trained model comprising a first neural network model, a second neural network model, and a third neural network model, wherein the first neural network model is trained to generate a feature vector for the main data, wherein the second neural network model is trained to perform one or more tasks for the main data, and wherein the third neural network model is trained to perform one or more tasks for the main data and/or the auxiliary data.

When the input data includes only main data, the second neural network model may be trained to perform one or more tasks for the input data, and, when the input data includes auxiliary data, the third neural network model may be trained to perform one or more tasks for the input data.

The performing may, when the input data includes only the main data, perform one or more tasks for the input data by using the first neural network model and the second neural network model, and when the input data includes the auxiliary data, perform one or more tasks for the input data by using the first neural network and the third neural network models.

The main data may be data generated from one or more brain MRI images of a cardiac arrest patient or a non-cardiac arrest patient.

The auxiliary data may be data generated from data comprising at least one of age information, gender information, blood test result information, cardiac arrest duration information, cardiac arrest cause information, body temperature information, consciousness information, and MRI equipment characteristic information of each of the cardiac arrest patient and the non-cardiac arrest patient.

The second neural network model and the third neural network model may be trained to analyze, as the one or more tasks, the input data with respect to at least one of a probability of death within a predetermined period, a probability of recovery of neurological function at a specific level or higher within the predetermined period, a lesion presence/absence probability, presence/absence of a specific lesion at a specific location, a probability of presence/absence of a specific disease, and an auxiliary task.

The second neural network model and the third neural network model may be trained to perform classification or regression analysis on the input data based on a type of a task performed on the input data.

### Advantageous Effects

The disclosed embodiments may use neural networks to determine the prognosis of a patient on brain MRI images without human intervention.

The disclosed embodiments may improve the performance of a neural network model for determining the prognosis of a cardiac arrest patient by using at least one of a diffusion weighted image (DWI) image, an apparent diffusion coefficient (ADC) image, and a fractional anisotropy (FA) image as input data and/or training data.

The disclosed embodiments may use brain MRI images of a non-cardiac arrest patient as training data to obtain training data of a neural network model for determining a prognosis of a cardiac arrest patient.

The disclosed embodiments may train all of the plurality of neural networks in the training process, but prevent overfitting by selectively using the neural network in the inference process.

### Brief Description of the Drawings

FIG. 1 is a block diagram for illustrating a brain magnetic resonance imaging (MRI) analysis device using a neural network, according to one embodiment.
FIG. 2 is a diagram for illustrating a process of generating input data by a brain MRI analysis device using a neural network, according to one embodiment.
FIG. 3 is an exemplary diagram for illustrating a structure of an example neural network model.
FIG. 4 is an exemplary diagram for illustrating a process by which an example training model is trained.
FIG. 5 is an exemplary diagram for illustrating a process by which an example training model is trained.
FIG. 6 is an exemplary diagram for illustrating a structure of an example neural network model.
FIG. 7 is a graph showing performance of a brain MRI analysis device using a neural network, according to one embodiment.
FIG. 8 is a flowchart for illustrating a brain magnetic resonance imaging (MRI) analysis method using a neural network, according to one embodiment.
FIG. 9 is a flowchart for illustrating a brain MRI analysis method using a neural network, according to one embodiment.
FIG. 10 is a flowchart for illustrating a brain MRI analysis method using a neural network, according to one embodiment.

### Embodiments of the Invention

The terminology used herein is selected from general terms currently widely used as much as possible in consideration of functions, but may vary depending on the intention or practice of those skilled in the art, or the appearance of new technologies, etc. In addition, in a specific case, there is also a term arbitrarily selected by the applicant, and in this case, the meaning thereof will be described in that description section of the specification. Therefore, it should be understood that the terms used herein should be interpreted based on the substantial meaning of the terms and the content throughout the present specification, rather than the mere names of the terms.

The terms such as first, second, third are only used for the purpose of distinguishing one component from another. For example, a first component could be termed a second component or third component, a second component also could be termed a first component or third component and, similarly, a third component could also be termed a first component or second component, without departing from the scope of the present disclosure.

As used herein, the singular forms "a," "an," and "the" include plural referents unless the context clearly dictates otherwise. In this application, the terms such as "comprise" or "include", "have" are intended to represent the presence of the component described herein or a combination thereof, but do not preclude the possibility that other components or features may be present or added.

Furthermore, embodiments described herein may have aspects that are wholly in hardware, partly in hardware and partly in software, or wholly in software. In this specification, a "unit", a "module", a "device", a "server", a "system", or the like refers to a computer-related entity such as hardware, a combination of hardware and software, or software. For example, a unit, a module, a device, a server, or a system may refer to hardware constituting a part or all of a platform and/or software such as an application for driving the hardware.

The following describes the embodiments in detail with reference to the accompanying drawings and contents described in the accompanying drawings, but the scope to be claimed is not limited or restricted by the embodiments.

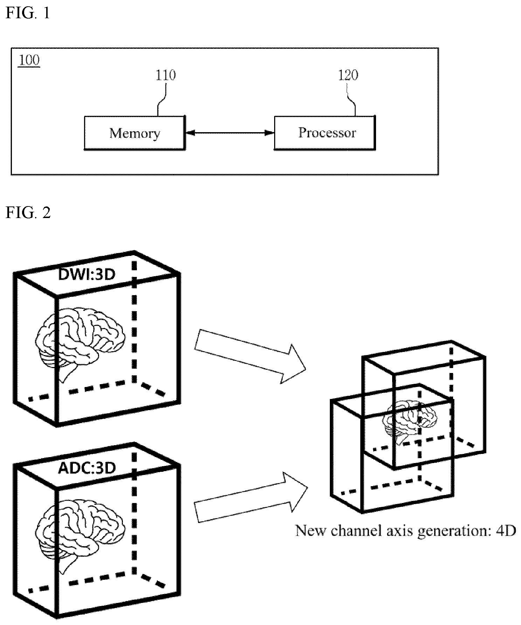

FIG. 1 is a block diagram for illustrating a brain magnetic resonance imaging (MRI) analysis device 100 using a neural network, according to one embodiment.

Referring to FIG. 1, a brain MRI analysis device 100 using a neural network includes a memory 110 and a processor 120.

Memory 110 may refer to hardware that stores information, such as data, in electrical or magnetic form for access by processor 120 and the like. For example, the memory 110 may be implemented by at least one hardware from among a non-volatile memory, a volatile memory, a flash memory, a hard disk drive (HDD) or solid-state drive (SSD), a RAM, a ROM, and the like. At least one instruction or module required for an operation of the brain MRI analysis device 100 or the processor 120 using a neural network, may be stored in the memory 110. Here, the instruction may be a code unit indicating an operation of the brain MRI analysis device 100 or processor 120 using a neural network, and may be written in a machine language that is a language understandable by a computer. A module may be an series of instruction set that performs a specific task of a task unit.

Data that is information in units of bits or bytes that may represent characters, numbers, images, etc., may be stored in the memory 110. For example, one or more neural network models may be stored in the memory 110. Here, each of the one or more neural network models may be a model that has been trained to predict various tasks related to the prognosis of the patient. However, this is merely illustrative and is not limited thereto. The memory 110 is accessed by the processor 120, and reading/writing/modifying/deleting/updating, etc., instructions, modules, or data may be performed by the processor 120.

The processor 120 generally controls the operation of the brain MRI analysis device 100 using the neural network. Specifically, the processor 120 may be connected to each configuration of brain MRI analysis device 100 using the neural network to generally control the operation of the brain MRI analysis device 100 using the neural network. For example, the processor 120 may be connected to a configuration such as the memory 110 to control the operation of the brain MRI analysis device 100 using the neural network.

The processor 120 may be implemented as a digital signal processor (DSP), a microprocessor, or a time controller (TCON). However, this is illustrative, and may include or be defined by one or more of a central processing unit (CPU), a micro controller unit (MCU), a micro processing unit (MPU), a controller, an application processor (AP), a communication processor (CP), and an advanced RISC machine processor (ARM) processor. In addition, the processor 120 may be implemented as a system on chip (SoC) or a large scale integration (LSI) in which a processing algorithm is embedded, and also may be implemented in form of a field programmable gate array (FPGA).

The processor 120 may receive one or more brain MRI images so as to generate input data.

The brain MRI image may be an image of the inside of the brain taken using MRI equipment. The brain MRI image may include at least one of a diffusion weighted image (DWI) image, an apparent diffusion coefficient (ADC) image, and a fractional anisotropy (FA) image.

Specifically, DWI is one of MRI techniques for imaging the degree of self-diffusion of water molecules in tissues. Accordingly, the DWI image may be an image generated by non-invasively measuring molecular diffusion inside a tissue using DWI.

By ADC is meant a measure of the size of diffusion of water molecules in a tissue. That is, the ADC image may be an image generated by imaging the ADC calculated by using the DWI. On the other hand, ADCs are generally calculated using MRI measurements with DWI mode.

FA is a measure of how well water molecules migrate along the tracts of white matter. The FA image may be an image generated by imaging the FA.

The processor 120 may generate each array based on the input one or more brain MRI images. For ease of description, the arrays generated corresponding to the DWI image, the ADC image, and the FA image are defined to be referred to as a DWI array, an ADC array, and an FA array, respectively.

The processor 120 may generate a three-dimensional array each having a preset size based on one or more input brain MRI images. Specifically, the processor 120 may generate a three-dimensional array in a format of D×W×H based on the one or more input brain MRI images, respectively. Here, D (depth) may refer to the number of images, W (width) may refer to a number of pixels in a horizontal direction, and H (Height) may refer to a number of pixels in a vertical direction.

The processor 120 may resize each array to a preset size so as to unify the sizes of the DWI array, the ADC array, and the FA array. For example, the processor 120 may change each array to a size J×K×L.

According to one example, the processor 120 may generate input data including main data and/or auxiliary data.

Here, the main data may be a three-dimensional array generated based on the one or more input brain MRI images for the cardiac arrest patient or the non-cardiac arrest patient.

The auxiliary data may be data that supplementarily describes the main data. For example, the auxiliary data may include data in text format including personal information about the patient corresponding to the main data, test results, and the like. Specifically, the auxiliary data may be or include date generated from, data comprising at least one of age information, gender information, blood test result information, cardiac arrest duration information, cardiac arrest cause information, body temperature information, consciousness information, and MRI equipment characteristic information of each of the cardiac arrest patient.

The auxiliary data may be data dependent on the main data. For example, when the main data is a DWI array of a non-cardiac arrest patient, the auxiliary data may be data including personal information, test information, etc. of the non-cardiac arrest patient.

The processor 120 may input the input data into a neural network model so as to acquire output data.

Here, the neural network model may be supervised and trained from training data consisting of a brain MRI image labeled as a brain MRI image of a cardiac arrest patient and a brain MRI image labeled as a brain MRI images of a non-cardiac arrest patient.

For this reason, the neural network model may obtain a large amount of training data by utilizing normal brain MRI images of non-cardiac arrest patients as training data instead of MRI images of cardiac arrest patients without a brain injury, which is difficult to find in clinical settings.

In this case, it is preferable that the gender and the age of the cardiac arrest patient and the non-cardiac arrest patient, to be samples of the training data, match each other. Here, the term "match" means not only a numerical match, but also an age match between a cardiac arrest patient and a non-cardiac arrest patient even when they belong to the same classified age interval.

On the other hand, the ratio of the abnormal brain MRI image of the cardiac arrest patient to the normal MRI image of the non-cardiac arrest patient may be preferably 1:3, which is illustrative and is not necessarily limited to the above ratio.

Meanwhile, here, the cardiac arrest may mean to a state in which the movement of the heart is stopped without limitation to types such as out-of hospital cardiac arrest (OHCA) and in hospital cardiac arrest (IHCA). The processor 120 may input the input data into a neural network model so as to acquire output data.

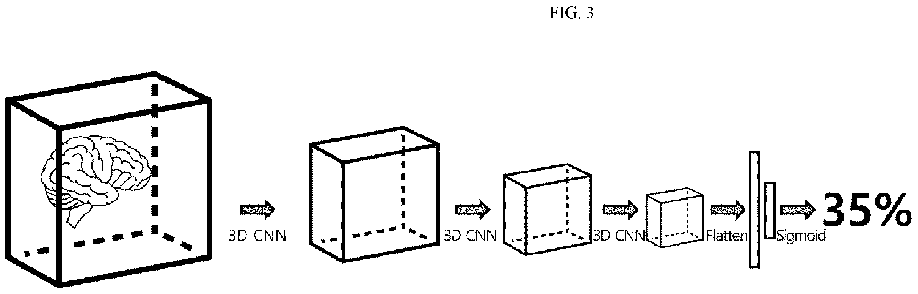

Here, the neural network model may be a model trained to perform a task for determining a prognosis of a cardiac arrest patient if input data is input into the model. Descriptions related to specific neural network models are described below in FIG. 3 to FIG. 6.

On the other hand, the neural network model is described as a model trained to perform a task for determining a prognosis of a cardiac arrest patient, but those skilled in the art of the disclosed embodiments may understand that the neural network model of the present disclosure may be used for predicting global ischemic injuries such as hypotension, hypoxia, carbon monoxide poisoning in addition to predicting cardiac arrest.

FIG. 2 is a diagram for illustrating a process of generating input data by a brain MRI analysis device 100 using a neural network, according to one embodiment.

Referring to FIG. 2, the processor 120 combines the DWI array and the ADC array so as to generate input data. In other words, the processor 120 combines the DWI array and the ADC array so as to generate the main data.

As shown in FIG. 2, when there are a plurality of types of the input images, the processor 120 may generate the input data by concatenating arrays generated corresponding to each of the input images along a new axis.

In other words, the processor 120 may generate new input data by superimposing a plurality of arrays having different kinds along a new axis. That is, the processor 120 may generate input data in a one-dimensional high array format by connecting different kinds of arrays.

For example, when a total of three types of images including a DWI image, an ADC image, and an FA image are input to the brain MRI analysis device 100 using the neural network according to one embodiment, the processor 120 may combine the DWI array, the ADC array, and the FA array so as to generate a four-dimensional array. If the input DWI, ADC, and FA arrays are of size J×K×L, the processor 120 may generate a 4-dimensional array of size 3 ×J×K×L as the input data.

On the other hand, the position of the dimension may be arbitrarily changed depending on the application method, and is not necessarily fixed.

FIG. 3 is an exemplary diagram for illustrating a structure of an example neural network model.

Referring to FIG. 3, a neural network model is a convolution neural network in which a plurality of 3D-CNNs are successively connected.

Referring to FIG. 3, the neural network model may be a convolution neural network in which three 3D-CNNs are repeatedly connected, and a fully connected layer (FC layer) is further connected.

Here, each 3D-CNN may be a neural network connected in the order of a normalization layer, an activation function, and a pooling layer after a convolution layer.

In this case, the hyperparameter and the structure of each 3D-CNN may be designed to be the same or different. For example, the hyperparameters of the first 3D-CNN may be kernel size: (3, 5, 5), stride: (2, 2, 2), padding: (1, 2, 2) number of input channels: 2, number of output channels: 4. The structure may be connected in the order of a convolution layer, a normalization layer (in particular, batch normalization), an activation function (in particular, ReLU), and a pooling layer (in particular, Max pooling).

The hyperparameters of the second 3D-CNN may be kernel size: (3, 5, 5), stride: (1, 2, 2), padding: (1, 2. 2), number of input channels: 4, number of output channels: 8. The structure may be connected in the order of convolution layer, normalization layer (in particular, batch normalization), activation function (in particular, ReLU) and flattening (Flatten), fully connected layer, dropout, activation function (in particular, ReLU), fully connected layer and activation function (in particular, sigmoid).

The hyperparameters of the third 3D-CNN may be kernel size: (3, 5, 5), stride: (1, 2, 2), padding: (1, 2, 2), the number of input channels: 8, the number of output channels: 16. The structure may be designed the same as the second 3D-CNN.

On the other hand, although the neural network model is shown to include three consecutive 3D-CNNs, this is illustrative and is not necessarily limited to the described number.

In addition, the structure and parameters of each 3D-CNN are illustrative and are not limited thereto, and it is intended that each structure and technique used may be arbitrarily changed.

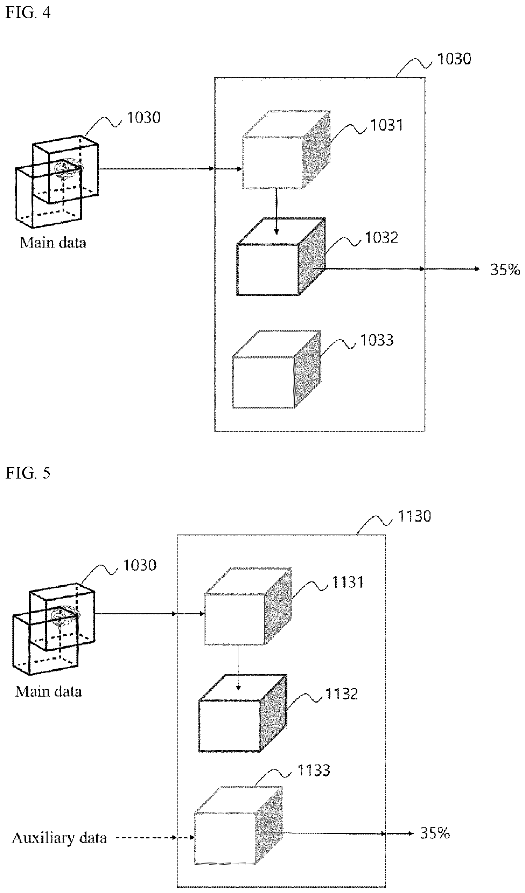

FIG. 4 is an exemplary diagram for illustrating a process by which an example training model 420 is trained.

Referring to FIG. 4, the training model 420 includes a first neural network model 421, a second neural network model 422, and a third neural network model 423.

Here, the first neural network model 421 is a model trained to generate a feature vector for the main data 410.

The second neural network model 422 is a model that has been trained to perform one or more tasks for the main data 410. Specifically, the second neural network model 422 may be trained to perform one or more tasks for the main data 410 by using the feature vector.

The third neural network model 423 is a model trained to perform one or more tasks for the main data 410 and/or the auxiliary data. Specifically, the third neural network model 423 may be trained to perform one or more tasks for the main data 410 and/or the auxiliary data by using the feature vector and the auxiliary data.

In particular, the second neural network model 422 and/or the third neural network model 423 may be a model trained to perform regression analysis and/or classification on the input data based on a type of a task performed on the input data.

For example, when the second neural network model 422 and/or the third neural network model 423 performs a main task for the input data, the second neural network models 422 and/or the third neural network models 423 may be models trained to classify the input data.

For another example, when the second neural network model 422 and/or the third neural network model 423 performs an auxiliary task for the input data, the second neural network models 422 and/or the third neural network models 423 may be models trained to perform classification or regression analysis the input data.

Here, the classification is a binary classification, and a sigmoid activation function is applied to the terminal of the neural network to output a value between 0 and 1, and regression analysis is predicting the value, and a separate activation function may not be required.

Referring to FIG. 4, when the input data includes main data 410, the main data 410 is input into the first neural network model 421. Then, the first neural network model 421 outputs a feature vector for the main data 410. Then, the second neural network model 422 receives the feature vector and performs one or more tasks for the input main data 410.

In other words, when the input data does not include auxiliary data, the brain MRI analysis device 100 using the neural network according to one embodiment may determine a neurological prognosis corresponding to the main data 410 by using only the first neural network model 421 and the second neural network model 422.

Meanwhile, when auxiliary data is not included in the input data in the training step, only the first neural network model 421 and/or the second neural network model 422 may be trained with respect to the main data 410. That is, in this case, the third neural network model 423 is excluded from training.

On the other hand, in FIG. 4, the second neural network model 423 is shown as outputting a neurological recovery probability of 35% as a prognosis determination, but the task for prognostic determination may include, in addition to the neurological recovery probability, various tasks such as probability of death within a predetermined period, a probability of recovery of neurological function at a specific level or higher within the predetermined period, a lesion presence/absence probability, presence/absence of a specific lesion at a specific location (e.g., an edema lesion, an ischemic lesion, a hemorrhagic lesion, a brain tumor, etc.), a probability of presence/absence of a specific disease (e.g, cerebral edema, cerebral infarction, cerebral hemorrhage, brain tumor, cerebral herniation, brain death, etc.) as a main task, but is not limited to the example shown in FIG. 4.

In addition, the one or more tasks may also include, as auxiliary tasks, various tasks such as predicting clinical findings and the presence/absence of diagnosis (e.g., brain injury blood test values such as NSE and S100, level of consciousness at the time of the test), predicting demographic information and underlying diseases (e.g, smoking history, age, previous cerebral infarction, etc.), predicting the contents implemented during data random augmentation (such as rotate, flip, crop, etc.), and the like.

On the other hand, according to an embodiment, it is described that the first neural network model 421 may be simultaneously trained with the second neural network model 422, but this is illustrative, and the first neural network models 421 may be pre-trained models that are not trained by input data.

FIG. 5 is an exemplary diagram for illustrating a process by which an example training model 530 is trained.

Referring to FIG. 5, the training model 530 includes a first neural network model 531, a second neural network model 532, and a third neural network model 533.

Here, the first neural network model 531, the second neural network model 532, and the third neural network model 533 are the same as the first neural network model 421, the second neural network model 422, and the third neural network model 423 of FIG. 4, and redundant descriptions thereof will be omitted.

Referring to FIG. 5, when the input data includes main data 510 and auxiliary data 520, the neural network model 533 performs one or more tasks for determining a prognosis of a cardiac arrest patient.

In other words, when the input data includes the main data 510 and the auxiliary data 520, the processor 120 preferably performs one or more tasks for determining the prognosis of the cardiac arrest patient by using only the third neural network model 533. That is, in inference, the brain MRI analysis device 100 using a neural network according to one embodiment may selectively use a neural network model according to whether auxiliary data is included in input data.

Meanwhile, in the training step under the same condition, the third neural network model 533 may be trained to perform one or more tasks for prognosis determination of the cardiac arrest patient by using the input main data 510 and/or the auxiliary data 520, and at the same time, the first neural network model 531 and/or the second neural network model 532 may also be trained in the same manner as in the case where the main data 510 is input.

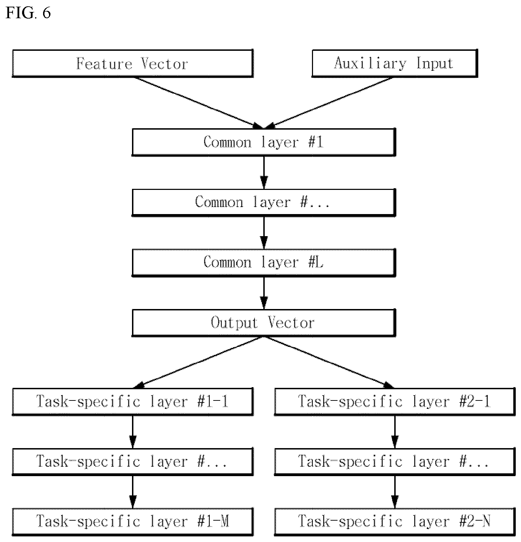

FIG. 6 is an exemplary diagram for illustrating a structure of an example training model 530.

Specifically, FIG. 6 may be an exemplary diagram of implementing an example of the third neural network model 533 of FIG. 5.

Referring to FIG. 6, structurally, the third neural network model 533 is a neural network model including one or more hidden layers as a multi-layer perceptron (MLP) structure.

The input data of the third neural network model 533 is a feature vector for the main data 510 output from the first neural network model 531 and the input auxiliary data 520.

In this case, the third neural network model 533 outputs an output vector by using the feature vector and the auxiliary data 520. Here, the output vector may be used to perform one or more tasks.

The third neural network model 533 is a task layer #1-1, #1-M, and may perform a main task by using M main task layers. On the other hand, the third neural network model 533 is an auxiliary task layer #2-1, #2-M, and may perform an auxiliary task by using M auxiliary task layers.

Meanwhile, it is desirable to have multiple main task layers and auxiliary task layers, but the number, type, and size are not limited.

On the other hand, in FIG. 5, the third neural network model 533 is shown in which the hidden layer is three MLPs, but this is illustrative, and a structure and a type of the third neural network model 533 are not limited thereto.

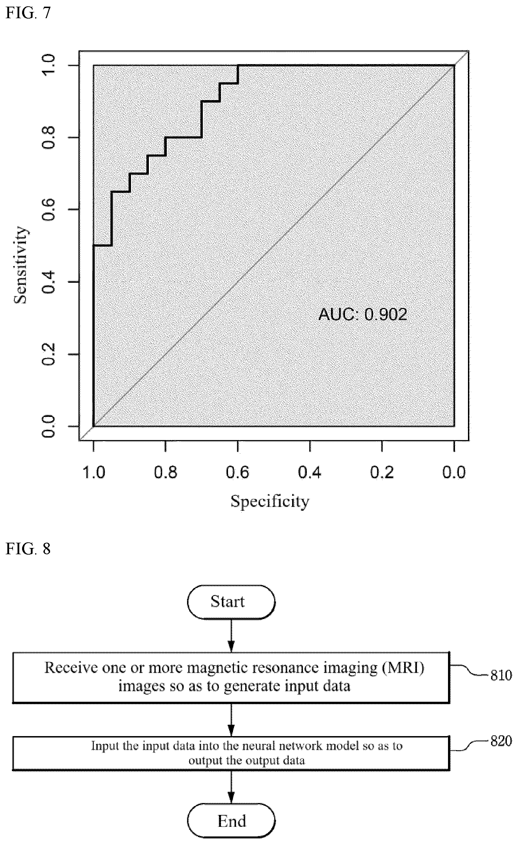

FIG. 7 is a graph measuring performance of a brain MRI analysis device 100 using a neural network, according to one embodiment.

FIG. 7 shows the performance of a pre-trained model including the first neural network models 421, 531 to the third neural network models 423, 533 as shown in FIG. 4 and FIG. 5.

Specifically, FIG. 7 shows a receiver operating characteristic (ROC) curve of the model of FIG. 7 for a test data set.

In addition, the model having the performance of FIG. 7 has the performance as shown in Table 1 below.

**Table 1**

| | AUC (95% CI) | Target Sensitivity (Threshold) | Sensitivity (95% CI) | Specificity (95% CI) | PPV (95% CI) | NPV (95% CI) |
|---|---|---|---|---|---|---|
| Train data set | 0.910 (0.857-0.964) | 1.000 (0.026) | 1.000 (1.000-1.000) | 0.516 (0.430-0.594) | 0.303 (0.270-0.342) | 1.000 (1.000-1.000) |
| | | 0.950 (0.056) | 0.963 (0.889-1.000) | 0.648 (0.563-0.734) | 0.366 (0.316-0.436) | 0.988 (0.963-1.000) |
| Test data set | 0.902 (0.814-0.991) | 1.000 (0.026) | 1.000 (1.000-1.000) | 0.500 (0.300-0.700) | 0.667 (0.588-0.769) | 1.000 (1.000-1.000) |
| | | 0.950 (0.056) | 1.000 (1.000-1.000) | 0.600 (0.400-0.800) | 0.714 (0.625-0.833) | 1.000 (1.000-1.000) |

FIG. 8 is a flowchart for illustrating a brain magnetic resonance imaging (MRI) analysis method using a neural network, according to one embodiment. Referring to FIG. 8, the method may be performed by the brain MRI analysis device 100 using the neural network as shown in FIG. 1. First, the brain MRI analysis device 100 using neural network according to one embodiment receives one or more brain MRI images so as to generate input data (810).

Then, the brain MRI analysis device 100 using the neural network according to one embodiment inputs the input data into the training model 420 so as to output the output data (820).

On the other hand, the training model 420 is trained to determine the neurological prognosis of the cardiac arrest patient if the input data is input to the model.

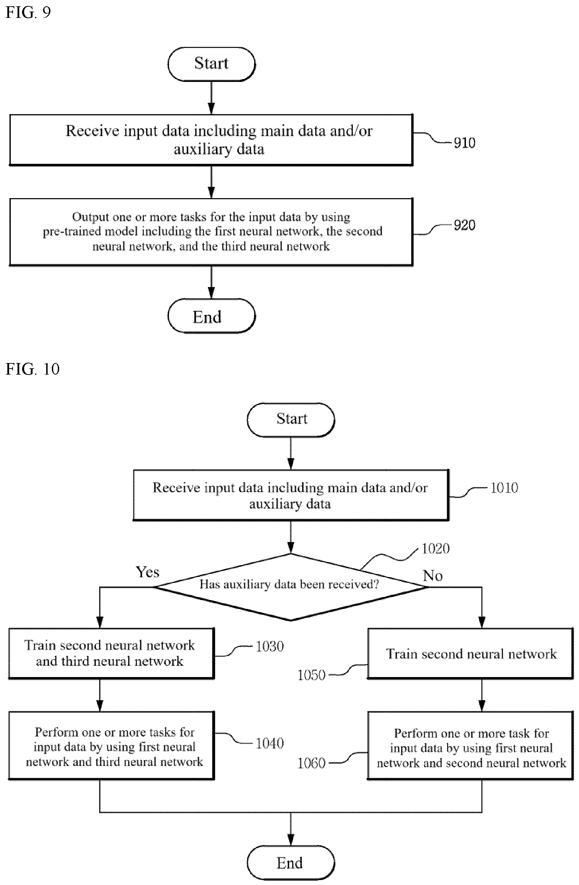

FIG. 9 is a flowchart for illustrating a brain MRI analysis method using a neural network, according to one embodiment.

Referring to FIG. 9, it may be performed by the brain MRI analysis device 100 using the neural network as shown in FIG. 1.

First, the brain MRI analysis device 100 using the neural network receives input data including main data 510 and/or auxiliary data 520 (910).

Thereafter, the brain MRI analysis device 100 using a neural network outputs one or more tasks for determining the prognosis of the cardiac arrest patient with respect to the input data by using the pre-trained model 530 including the first neural network model 531, the second neural network model 532, and the third neural network model 533 (920).

Meanwhile, the first neural network model 531 is trained to generate a feature vector for the main data 510, the second neural network model 532 is trained to perform one or more tasks for the main data 510, and the third neural network model 533 is trained to perform the one or more tasks for the main data 510 and/or the auxiliary data 520.

* FIG. 10 is a flowchart for illustrating a brain MRI analysis method using a neural network, according to one embodiment.

Referring to FIG. 10, the method may be performed by the brain MRI analysis device 100 using the neural network as shown in FIG. 1.

First, the brain MRI analysis device 100 using the neural network receives input data including main data 510 and/or auxiliary data 520 (1010).

Then, the brain MRI analysis device 100 using the neural network determines whether input data including the auxiliary data 520 has been received (1020).

When the brain MRI analysis device 100 using the neural network receives the auxiliary data 520 in which the auxiliary data 520 is included in the input data, one or more tasks for the input data are performed by using the third neural network model 533 (1040). Meanwhile, at the same time or before, the second neural network model 532 and the third neural network model 533 receive the auxiliary data 520 and are trained to perform one or more tasks (1030).

On the other hand, when the brain MRI analysis device 100 using the neural network does not include the auxiliary data 520 in the input data, that is, only the main data 410 is received, the brain MRI analysis device 100 using the neural network performs one or more tasks for the input data by using the first neural network model 421 and the second neural network model 422 (1060). Meanwhile, at the same time or before, the second neural network model 422 receives the main data 410 and is trained 1050 to perform one or more tasks.

The method shown in FIGS. 8 to 10 has been described with reference to the flowchart shown in the drawings. Although, for purposes of explanation, the method is shown and described as a series of blocks, the present disclosure is not limited by the order of the blocks, as some blocks may occur in different orders or concurrently with other blocks from that shown and described herein, and various other branches, flow paths, and orders of blocks may be implemented which achieve the same or similar results. Moreover, not all shown blocks may be required for implementation of the methods described herein.

Furthermore, the method according to an embodiment of the present disclosure may be implemented in the form of a computer program for performing a series of processes, and the computer program may be recorded on a computer-readable recording medium. Examples of the computer-readable recording medium include magnetic media such as a hard disk, a floppy disk, and a magnetic tape, optical recording media such as a CD-ROM and a DVD, magneto-optical media such as a floptical disk, and hardware devices specially configured to store and perform program instructions such as a ROM, a RAM, and a flash memory.

While the foregoing has been described with reference to embodiments, it will be understood by those skilled in the art that various modifications and variations can be made to the disclosure without departing from the spirit and scope of the disclosure as described in the following claims.

### Industrial Applicability

The brain MRI analysis device and method using a neural network according to one embodiment are applicable to the digital medical industry by determining a prognosis of a patient on a brain MRI image without human intervention using a neural network.

## Claims

1. A brain magnetic resonance imaging (MRI) analysis device using a neural network, comprising:
a memory for storing a neural network model; and
a processor, which is connected to the memory so as to control the analysis device,
wherein the processor receives one or more brain MRI images so as to generate input data, and inputs the input data into the neural network model so as to acquire output data, and
wherein the neural network model is trained to determine neurological prognosis of a cardiac arrest patient if the input data is input into the neural network model.

2. The brain MRI analysis device using the neural network of claim 1, wherein
the one or more brain MRI images comprise at least one of a diffusion weighted image (DWI) image, an apparent diffusion coefficient (ADC) image, and a fractional anisotropy (FA) image.

3. The brain MRI analysis device using the neural network of claim 2, wherein
the processor:
generates each of the DWI image, the ADC image, and the FA image as a three-dimensional array of a preset size, and
when there are a plurality of types of the brain MRI images, generates the input data by concatenating a plurality of arrays of the DWI array, the ADC array, and the FA array along a new axis.

4. The brain MRI analysis device using the neural network of claim 1, wherein
the neural network model is successively connected in a plurality of 3D-convolution neural networks (3D-CNN).

5. The brain MRI analysis device using the neural network of claim 4, wherein
the neural network model is a convolution neural network in which each of the plurality of 3D-CNNs is successively connected in the order of a normalization layer, an activation function, and a pooling layer after a convolution layer, followed by a fully connected layer (FC layer).

6. The brain MRI analysis device using the neural network of claim 1, wherein
the neural network model is supervised and trained from training data consisting of a brain MRI image labeled as a brain MRI image of a cardiac arrest patient and a brain MRI image labeled as a brain MRI image of a non-cardiac arrest patient.

7. A brain magnetic resonance imaging (MRI) analysis method using a neural network, performed in a brain MRI analysis device using a neural network comprising:
one or more processors, and
a memory storing one or more neural network models executed by the one or more processors, the method comprising:
receiving one or more brain MRI images so as to generate input data; and
inputting the input data into the neural network model so as to output the output data,
wherein the neural network model is trained to determine neurological prognosis of a cardiac arrest patient if the input data is input into the neural network model.

8. The brain MRI analysis method using the neural network of claim 7, wherein
the one or more brain MRI images comprise at least one of a diffusion weighted image (DWI) image, an apparent diffusion coefficient (ADC) image, and a fractional anisotropy (FA) image.

9. The brain MRI analysis method using the neural network of claim 8, wherein
the processor:
generates each of the DWI image, the ADC image, and the FA image as a three-dimensional array of a preset size, and
when there are a plurality of types of the brain MRI images, generates the input data by concatenating a plurality of arrays of the DWI array, the ADC array, and the FA array along a new axis.

10. The brain MRI analysis method using the neural network of claim 7, wherein
the neural network model comprises a plurality of 3D-convolution neural networks (3D-CNN) connected in succession.

11. The brain MRI analysis method using the neural network of claim 10, wherein
the neural network model is a convolution neural network in which each of the plurality of 3D-CNNs is successively connected in the order of a normalization layer, an activation function, and a pooling layer after a convolution layer, followed by a fully connected layer (FC layer).

12. The brain MRI analysis method using the neural network of claim 7, wherein
the neural network model is supervised and trained from training data consisting of a brain MRI image labeled as a brain MRI image of a cardiac arrest patient and a brain MRI image labeled as a brain MRI image of a non-cardiac arrest patient.

13. A brain magnetic resonance imaging (MRI) analysis device using a plurality of neural network models, comprising:
a memory for storing a plurality of neural network models; and
a processor, which is connected to the memory so as to control the analysis device,
wherein the processor receives input data comprising main data and/or auxiliary data, and performs one or more tasks for the input data to determine prognosis of a cardiac arrest patient by using a pre-trained model comprising a first neural network model, a second neural network model, and a third neural network model,
wherein the first neural network model is trained to generate a feature vector for the main data,
wherein the second neural network model is trained to perform one or more tasks for the main data, and
wherein the third neural network model is trained to perform one or more tasks for the main data and/or auxiliary data.

14. The brain MRI analysis device using the neural network of claim 13, wherein
when the input data comprises main data, the second neural network model is trained to perform one or more tasks for the input data, and
when the input data comprises auxiliary data, the third neural network model is trained to perform one or more tasks for the input data.

15. The brain MRI analysis device using the neural network of claim 13, wherein
the processor:
when the input data comprises only the main data, performs one or more tasks for the input data by using the first neural network model and the second neural network model, and
when the input data comprises the auxiliary data, performs one or more tasks for the input data by using the first neural network model and the third neural network model.

16. The brain MRI analysis device using the neural network of claim 13, wherein
the main data is data generated from one or more brain MRI images of a cardiac arrest patient or a non-cardiac arrest patient.

17. The brain MRI analysis device using the neural network of claim 16, wherein
the auxiliary data is data generated from data comprising at least one of age information, gender information, blood test result information, cardiac arrest duration information, cardiac arrest cause information, body temperature information, consciousness information, and MRI equipment characteristic information of each of the cardiac arrest patient and the non-cardiac arrest patient.

18. The brain MRI analysis device using the neural network of claim 13, wherein
the second neural network model and the third neural network model are trained to analyze, as the one or more tasks, the input data with respect to at least one of a probability of death within a predetermined period, a probability of recovery of neurological function at a specific level or higher within the predetermined period, a lesion presence/absence probability, presence/absence of a specific lesion at a specific location, a probability of presence/absence of a specific disease, and an auxiliary task.

19. The brain MRI analysis device using the neural network of claim 18, wherein
the second neural network model and the third neural network model are trained to perform classification or regression analysis on the input data based on a type of a task performed on the input data.

20. A brain magnetic resonance imaging (MRI) analysis method using a neural network, performed in a brain MRI analysis device using a neural network comprising:
one or more processors, and
a memory for storing one or more neural network models executed by the one or more processors,
the method comprising:
receiving input data comprising main data and/or auxiliary data; and
performing one or more tasks for the input data to determine a prognosis of a cardiac arrest patient by using a pre-trained model comprising a first neural network model, a second neural network model, and a third neural network model,
wherein the first neural network model is trained to generate a feature vector for the main data,
wherein the second neural network model is trained to perform one or more tasks for the main data, and
wherein the third neural network model is trained to perform one or more tasks for the main data and/or the auxiliary data.

21. The brain MRI analysis method using the neural network of claim 20, wherein
when the input data comprises only main data, the second neural network model is trained to perform one or more tasks for the input data, and
when the input data comprises auxiliary data, the third neural network model is trained to perform one or more tasks for the input data.

22. The brain MRI analysis method using the neural network of claim 20, wherein
the performing:
when the input data comprises only the main data, performs one or more tasks for the input data by using the first neural network model and the second neural network model, and
when the input data comprises the auxiliary data, performs one or more tasks for the input data by using the first neural network and the third neural network models.

23. The brain MRI analysis method using the neural network of claim 20, wherein
the main data is data generated from one or more brain MRI images of a cardiac arrest patient or a non-cardiac arrest patient.

24. The brain MRI analysis method using the neural network of claim 23, wherein
the auxiliary data is data generated from data comprising at least one of age information, gender information, blood test result information, cardiac arrest duration information, cardiac arrest cause information, body temperature information, consciousness information, and MRI equipment characteristic information of each of the cardiac arrest patient and the non-cardiac arrest patient.

25. The brain MRI analysis method using the neural network of claim 20, wherein
the second neural network model and the third neural network model are trained to analyze, as the one or more tasks, the input data with respect to at least one of a probability of death within a predetermined period, a probability of recovery of neurological function at a specific level or higher within the predetermined period, a lesion presence/absence probability, presence/absence of a specific lesion at a specific location, a probability of presence/absence of a specific disease, and an auxiliary task.

26. The brain MRI analysis method using the neural network of claim 25, wherein
the second neural network model and the third neural network model are trained to perform classification or regression analysis on the input data based on a type of a task performed on the input data.
